# EUROPEAN PATENT SPECIFICATION

(11) **EP 4 463 131 B1**
(45) Date of publication and mention of the grant of the patent: **07.10.2026**
(21) Application number: 23700743.0
(22) Date of filing: 11.01.2023
(51) Int. Cl.: A61K 8/26, A61K 8/73, A61Q 19/08, A61Q 19/00

(54) **COMPOSITION**
ZUSAMMENSETZUNG
COMPOSITION

(30) Priority: 13.01.2022 GB 202200421; 23.09.2022 GB 202213932
(43) Date of publication of application: 20.11.2024
(73) Proprietor: Givaudan SA, 1214 Vernier (CH)
(72) Inventor: SCANDOLERA, Amandine, 51110 Pomacle (FR); DE TOLLENAERE, Morgane, 51110 Pomacle (FR); REYNAUD, Romain, 31400 Toulouse (FR)
(74) Representative: Global Patents
(86) International application number: PCT/EP2023/050544
(87) International publication number: WO 2023/135170

(56) References cited:
- CN-A- 105 213 267
- CN-A- 111 494 252
- JP-A- 2015 189 707
- US-A- 5 165 915

## Description

This disclosure relates to a method of skin treatment, and to compositions for effecting such treatment.

Hyaluronic acid is a naturally-occurring glycosaminoglycan found throughout the body's connective tissue. It is the main component of what gives skin structure, and is responsible for providing a plump and hydrated look. As a result, there have emerged over recent years a substantial number of hyaluronic acid-based skin treatment preparations. For example CN 105 213 267 A discloses an anti-ageing cream comprising medium and high molecular weight (Mw) fraction of hyaluronic acid.

For the best results, it is desired that the hyaluronic acid penetrate deeply into the skin. With conventional skin creams, this has not always been possible. It has now been surprisingly found that a particular delivery means can provide unusually deep skin penetration, with concomitant improvements in skin condition. There is therefore provided a skin treatment preparation, comprising hyaluronic acid having a molecular weight of from 10-1000KDa, absorbed into an activated smectite clay by blending under conditions of high shear, the hyaluronic acid - clay combination having a non-pore specific surface of from 3-10 M²/g and a particle size (VSSA) of from 250-500nm.

There is additionally provided a method of providing hyaluronic acid to the skin, the hyaluronic acid comprising part of a hyaluronic acid-clay combination as hereinabove described.

There is additionally provided an activated smectite clay into which is blended hyaluronic acid under conditions of high shear, the hyaluronic acid - clay blend having a non-pore specific surface of from 3-10 M²/g and a particle size (VSSA) of from 250-500nm.

Volume Specific Surface-Area (VSSA) is an integral measurement method that provides an indirect representation of particle size.

Smectite clays are a group of platy phyllosilicate minerals of 2:1 layer and a layer charge from about -0.2 - - 0.6 per formula unit. They have a large specific surface area and exhibit a high degree of swelling in water. A particular example of a smectite clay is montmorillonite, more particularly bentonite.

Combinations of hyaluronic acid ("HA") and clays are known and commercially available as part of skin-care treatments. However, it has been surprisingly found that combinations as hereinabove defined can penetrate much more deeply into the skin than can simple mixtures of HA and clay.

The parameters of the HA-clay blends of this disclosure cannot be achieved by simple mixing. The preparation of the HA-clay blend of the disclosure is carried out by applying a very high shear in a rotor/stator mixer, similar to the shear produced in an extruder reactor. Any such mixer may be used, for example ball mills, ribbon blenders, paddle blenders, screw blenders and double cone blenders.

Prior to blending, the smectite clay is activated. This may be any kind of activation known to the art, a typical example being acid activation, as described, for example by Maged et al in Environmental Science and Pollution Research, 27, pp. 32980-32997 (2020). After drying and sieving, the activated smectite clay is subjected to high shear. The shearing time will depend on the particular materials and shearing method used, but the skilled person can readily ascertain by simple experimentation a suitable duration in each case. A typical, non-limiting shearing time will be from 15 minutes to 2 hours, particularly from 30 minutes to 1 hour.

It is believed, without restricting the disclosure in any way, that pressure and shear forces have the ability to increase interactions between the clay and the HA. Moreover, under such combination of pressure (>1bar) and shear forces, it is believed that the lamellar structure of the clay opens up, making hydroxyl groups on the platelet edges of the clay more available. It is believed, again without restricting the disclosure in any way, that the interactive mechanism is through hydrogen bonding between the hydrophilic moieties in HA (such as carboxylic acid or hydroxyl groups) and hydroxyl groups on the clay.

It is believed, again without restricting the disclosure in any way, that interaction between HA and clay confers on the HA a strongly anionic character with lower electrical potential as measured by zeta potential, which is significantly different from that of the HA alone. This electrical behavior is believed to give better skin affinity with the viable epidermis layer leading to skin penetration of HA, whereas the HA alone cannot penetrate at all. It is known that there is a natural gradient of pH in the skin, leading to the existence of more positive charges at the surface. As a result, hyaluronic acid applied alone tends to stay at or near the surface of the skin.

In particular embodiments, the specific surface is from 4-9, more particularly from 5-8 m²/g In other particular embodiments, the particle size (VSSA) is from 280-450, more particularly from 300-430nm.

These figures are lower (for specific surface area) and higher (for particle size) because of the method of production of the HA-clay blends of this disclosure.

The activated smectite clay may be used unmodified, but in a particular embodiment, it may be modified with a fatty substance, to enhance absorption into the skin. Typical substances include oils and hydrocarbon waxes of animal or vegetable or mineral origin, silicone oils, or their mixture. Particular examples of hydrocarbon-based modifiers include vegetable and animal oils and fats, more particularly triglycerides; synthetic ethers; linear or branched hydrocarbons, of mineral or synthetic origin, such as petroleum jelly; synthetic esters such as isopropyl myristate and fatty alcohol benzoates; heptanoates, octanoates, decanoates or ricinoleates of alcohols or polyalcohols; hydroxylated esters such as isostearyl lactate, esters of polyols; fatty alcohols such as octyldodecanol; higher fatty acids such as linolenic acid; silicone oils of polymethylsiloxane type and mixtures thereof.

Examples of vegetable waxes include carnauba, candelilla, jojoba wax or any other vegetable compound consisting of an ester of ethylene glycol and of two fatty acids or of a monester of fatty acid and of long-chain alcohol; animal waxes such as beeswax. Other fatty substances and lipophilic additives include essential oils; natural aromatic compounds and lipophilic syntheses; natural or synthetic fat-soluble vitamins such as tocopherol or alphatocopheryl acetate.

The proportion of fatty substances in the clay typically varies from 0.05 to 14.5% by weight.

The hyaluronic acid of the disclosure has a molecular weight of from 10-1000 kDa, particularly from 20-1500 kDa, more particularly from 50-1400 kDa, more particularly from 100-1100 kDa, and even more particularly from 300-1000 kDa. It may be added as the acid, or as the alkali metal salt (typically sodium) with a suitable acid, such as citric acid, for generation of the acid

The modified clay may be made into a skin treatment preparation by any known means. The preparation may contain all the normal ingredients of such preparations in artrecognised proportions. Non-limiting examples include vitamins, antioxidants, thickeners, trace elements, softeners, sequestering agents, perfumes, basifying or acidifying agents, preservatives, UV filters, hydrophilic or lipophilic active ingredients and mixtures thereof. The hyaluronic acid is present in such a preparation in the weight proportion of from 5-15%, particularly from 8-12%, more particularly from 9-11%.

The surprising effect of this particular combination of the smectite clay and hyaluronic acid is that it penetrates particularly deeply into the skin. It is believed, with restricting the disclosure in any way, that the clay modifies the zeta-potential of the hyaluronic acid, making the combination more negatively charged. It is known that there is a natural gradient of pH in the skin, leading to the existence of more positive charges at the surface. As a result, hyaluronic acid tends to stay at or near the surface of the skin. However, the negative charge imparted by the clay allows the deeper penetration into the skin, with resulting beneficial effects.

An additional unexpected and surprising benefit is the enhancement of a feeling of well-being among people to whose skin a preparation according to the disclosure has been applied. This benefit has been scientifically verified by a testing procedure further described in the examples.

The disclosure is further described with reference to the following non-limiting figures and examples.
Figure 1 is a graphical representation of the depth penetration into the skin of hyaluronic acid when applied alone, and in a simple blend with clay, as available commercially.
Figure 2 is a repeat of Figure 1, but with the simple clay-hyaluronic acid blend replaced by a hyaluronic acid-clay blend as described in this disclosure.

### Example 1

### Testing in a skin cream; comparative example

### (a) Preparation of hyaluronic acid-clay blend

The hyaluronic acid- clay blend was prepared by taking a commercially-available bentonite clay, activating it by the method described by Maged et al in Environmental Science and Pollution Research, 27, pp. 32980-32997 (2020), drying and sieving it using a 100µm filter, adding to it sodium hyaluronate and then shearing this mixture in a high-shear ribbon blender for one hour

### (b) Preparation of test skin creams

Two test skin creams were prepared by blending the following ingredients

| | Cream 1 | Cream 2 |
|---|---|---|
| water | 87.1 | 88.1 |
| Emulium^{™} Delta MD* | 5.0 | 5.0 |
| Isodecyl neopentonate | 4.5 | 4.5 |
| HA clay | - | 1 |
| glycerin | 1 | 1 |
| phenoxyethanol | 1 | 1 |
| dimethicone | 0.3 | 0.3 |
| fragrance | 0.1 | 0.1 |

| | | |
|---|---|---|
| *Commercially-available emulsifier (ex Gattefossé) | | |

### (c) Testing of creams

The test subjects were 20 women in the age range 35-55, all of whom had dry skin and who had shallow wrinkles and crow's feet. They were split into two groups, designated Group A and Group B. Group A applied Cream 1, whereas Group B applied Cream 2.

All subjects applied the creams twice daily, morning and evening, on the face. The skin was examined immediately prior to first application, then at 1 hour, 6 hours, 7 days and 28 days. Examination was by means of a Visia^{™} CR2.3 visual scanner (ex Canfield Scientific), which measured the area of the crow's feet. The results are shown in the following table:

| | % reduction in crow's feet area after... | | | |
|---|---|---|---|---|
| Test subjects | 1hour | 6 hours | 7 days | 28 days |
| Group A | 1.1 | 5 | 6.9 | 4.6 |
| Group B | 18.5 | 21.5 | 16.1 | 27.3 |

Thus, the improvement provided by Cream 1 is 17.4% after 1 hour, 16.5% after 6 hours and 22.7% after 28 days.

### Example 2

Demonstration of the skin penetration of hyaluronic acid when used in a blend prepared according to this disclosure.

The following materials were tested:
- Untreated clay ("untreated")
- Hyaluronic acid (HA)
- HA + clay ("simple mixture")
- HA-clay blend according to this description ("HA Clay")

HA was used in distilled water at 1% by weight, and both simple mixture and HA Clay were used at 10% in distilled water, both containing 1% by weight of hyaluronic acid.

Human skin explants from a 47 years old donor were prepared and kept in survival medium (MIL215001, Biopredic) for 24 hours at 37°C and 5% CO₂. The next day, HA Clay and HA were topically applied and incubated for 8 hours at 37°C and 5% CO₂ before skin penetration analysis. An untreated sample was used as a control. After the end of incubation, the skin surface was cleaned in order to eliminate any excess of the product. The skin explants were then frozen at -80°C and cut longitudinally using a cryotome with a thickness of 20µm. For each explant, 3 tissue sections were selected and deposited on a CaF₂ support for Raman imaging analysis for a total of 9 Raman images per condition. 3 other adjacent sections of 7µm thickness were prepared for an Hematoxylin & Eosin staining.

The Raman images had a size of Y: 10µm / X: 100µm with a step of 5µm in X and 5µm in Y. Each Raman image has 3Y spectra and 21X spectra (63 spectra per image).
- Laser wavelength: 600nm
- Objective: 100 X, long focal length with a numerical aperture 0.75
- Acquisition time: 25 seconds
- Accumulation: 1X
- Spectral range: 400 to 4000 cm⁻¹
- Gratting: 950T
- Confocal Hole: 300µm
- Slit width: 150µm (spectral resolution 6.5 cm⁻¹)
- Step in X: 5µm, Step in Y: 5µm

In order to ensure reproducibility of the measurements, before each use, the Raman spectrometer was calibrated with silicon which gives a Raman peak at 520.7 cm⁻¹. Continuous control of the laser power at the sample level was achieved.

A pre-processing of Raman images was made by eliminating aberrant spectra (fluorescence, burning, saturation), correcting the baseline, applying a spectral smoothing and despike and a spectral normalization.

The processing of corrected data maps was performed by using software based on least squares fitting method that operates with Matlab software. This method involved mathematical modelling of reference spectra in the overall spectral image to determine the contribution and distribution of these spectra within the image. In this study, the average spectra of hyaluronic acid and clay were used as reference spectra.

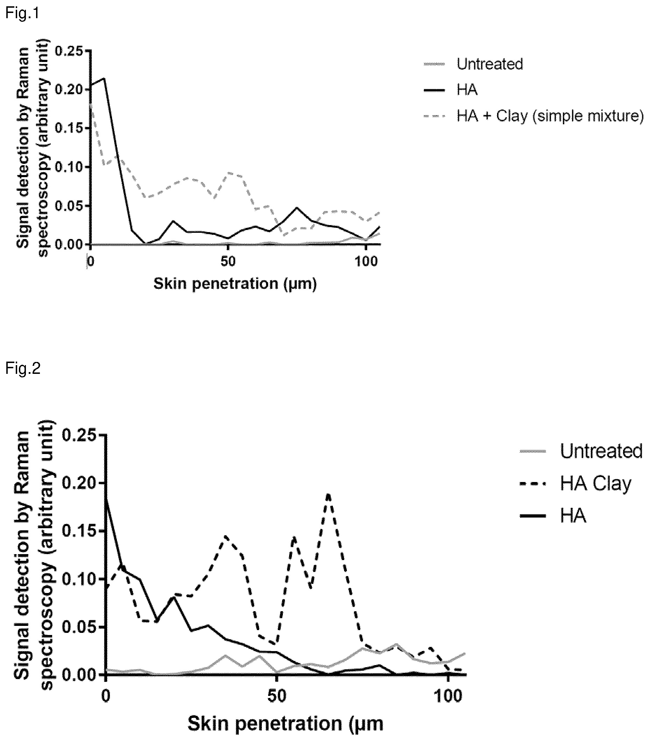

It can be seen from Figures 1 and 2 that the penetration into the skin of the HA Clay is considerably deeper than either the HA alone or the simple mixture. The clay alone has very little penetration into the skin, showing conclusively that the HA Clay combination produced according to this disclosure gives much deeper skin penetration

### Example 3

Demonstration of an enhanced feeling of well-being experienced by recipients of a composition as hereabove described.

### (a) Preparation of skin cream

Two skin creams were prepared, one containing a hyaluronic acid/clay blend prepared according to Example 1, the other without the blend and used as a placebo. The formulae are shown below, Cream 3 being the cream with the hyaluronic acid/clay blend and Cream 4 the placebo

| | | Cream 3 | Cream 4 |
|---|---|---|---|
| A | Water | To 100% | To 100% |
| | cross-linked polyacrylic acid homopolymer¹ | 0.5 | 0.5 |
| | 1,2-hexanediol/1,2-octanediol blend¹ | 0.3 | 0.3 |
| | phenoxyethanol | 0.5 | 0.5 |
| B | 30.5% sodium hydroxide | To pH7 | To pH7 |
| C | Caprylocaproyl Macrogolglycerides² | 1 | 1 |
| | C8/C10 medium-chain triglycerides³ | 5 | 5 |
| D | Hyaluronic acid/clay | 1 | 0 |
| | water | 4 | 0 |
| Viscosity⁵ | | 15,000 | 8,000 |

| | | | |
|---|---|---|---|
| 1 Carbopol^{™} ETD 2050 ex Lubrizol 2 Symdiol^{™} 68, ex Symrise 3 Dubcare^{™} GPE 810 ex Stearinerie Dubois 4 Miglyol^{™} 812N ex IOI Oleo GmbH 5 viscosity in mPa.s (Brookfield DVIII Ultra, Spindle F, speed 12 @ 20°C) | | | |

### (b) Testing of skin creams

Testing was performed on 59 subjects, 42 women and 17 men, average age 38, all ordinary members of the public. Testing was double-blind, in that the testers also did not know which of Cream 3 or Cream 4 was being presented to the test subject.

Testing was performed by the subjects applying the product on the hands on 2 consecutive days. The test subjects were asked to complete the questionnaire shown on the following page before and after the testing. The questionnaire was presented on a computer screen and each question was accompanied by a slider that could be moved to present a position between "Not at all" (value = 0) and "Very" (value =10). A representation of the screen is shown below.

| | [Not at all] [Very] |
|---|---|
| How **MOTIVATED** do you feel RIGHT NOW? | 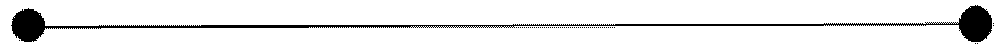 |
| How **INTERESTED** do you feel RIGHT NOW? | 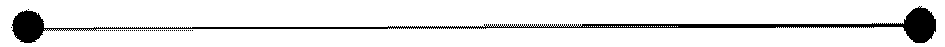 |
| How **SATISFIED** do you feel RIGHT NOW? | 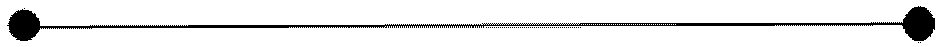 |
| How **OPTIMISTIC** do you feel RIGHT NOW? | 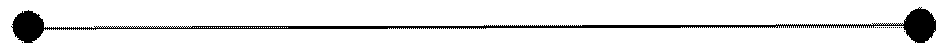 |
| How **FRUSTRATED** do you feel RIGHT NOW? | 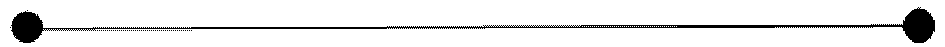 |
| How **INVIGORATED** do you feel RIGHT NOW? | 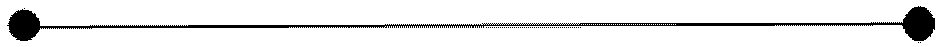 |
| How **FATIGUED** do you feel RIGHT NOW? | 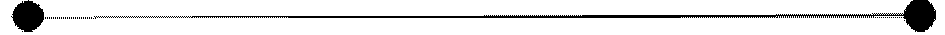 |
| How **RESTLESS** do you feel RIGHT NOW? | 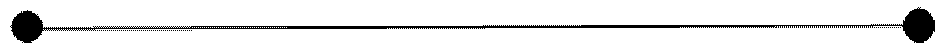 |
| How **HAPPY** do you feel RIGHT NOW? | 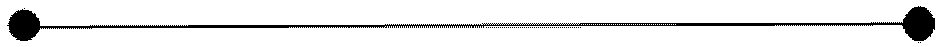 |
| How **BORED** do you feel RIGHT NOW? | 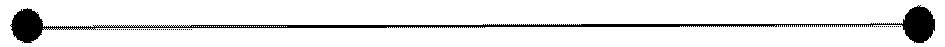 |
| How **CALM** do you feel RIGHT NOW? | 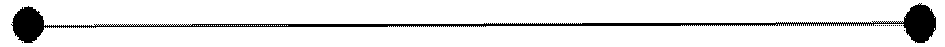 |
| How **RELAXED** do you feel RIGHT NOW? | 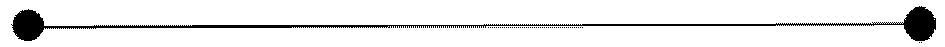 |
| How **EXCITED** do you feel RIGHT NOW? | 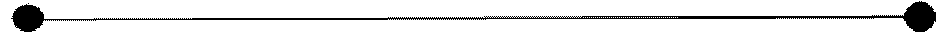 |
| How **SAD** do you feel RIGHT NOW? | 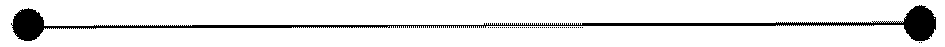 |
| How **MENTALLY ALERT** do you feel RIGHT NOW? | 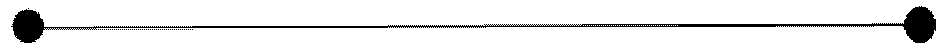 |
| How **STRESSED** do you feel RIGHT NOW? | 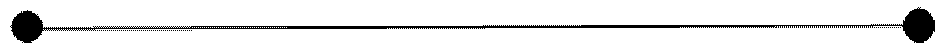 |
| How **ANXIOUS** do you feel RIGHT NOW? | 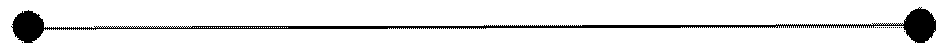 |
| How **PATIENT** do you feel RIGHT NOW? | 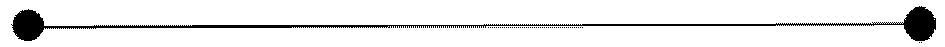 |

A well-being metric was used for this study to measure the effects of the test cream on the well-being of test subjects. The well-being metric, described in detail in International Publication WO 2020/165463, to which reference may be made, was defined by applying experimental psychology and unsupervised clustering of verbal attributes of well-being in order to identify the most relevant dimensions to assess well-being. The well-being attributes take into account various aspects of well-being, such as affective, eudaimonic, social, and physical aspects. These aspects may have both affective (emotional) and cognitive (rational) components.

The weighting of the well-being attributes was determined as follows:
a) Having one or more human subject(s) assess their well-being in the absence of a test cream by providing a well-being score *wbᵢ* for each of the WB-18 attributes;
b) Applying a factor analysis, in particular a principal component analysis, on the well-being scores obtained in step a), in order to determine the impact of each well-being attribute on the overall well-being, resulting in *M* well-being factors *Fⱼ*;
   i) wherein each of the well-being factors *Fⱼ* has a variance *vⱼ* expressed as a percentage of the sum of the variances of all well-being factors;
   ii) wherein each of the well-being factors *Fⱼ* comprises a finite number *nⱼ* of well-being attributes, having a loading *lᵢ*, expressed as a percentage of the sum of the *nⱼ* loadings ; and
   iii) wherein none of the well-being attributes appears in more than one well-being factor; and
c) Calculating the well-being score in the absence of the test perfume according to the following equation ${WB}_{0} = {\sum }_{1}^{M} {v}_{j} {F}_{j , 0} = {\sum }_{1}^{M} {v}_{j} {\sum }_{1}^{{n}_{j}} {l}_{i} {wb}_{i}$
The principal component analysis revealed that:
- the well-being attributes "not anxious", "not sad", "not restless", "not frustrated", "not stressed" are associated with a first well-being factor *F*₁ having a variance *v*₁;
- the well-being attributes "happy", "optimistic", "excited", "satisfied", "motivated", "invigorated" are associated with a second well-being factor *F*₂ having a variance *v*₂;
- the well-being attributes "interested" and "not bored" are associated with a third well-being factor *F*₃ having a variance *v*₃;
- the well-being attributes "not fatigued" and "mentally alert" are associated with a fourth well-being factor *F*₄ having a variance *v*₄;
- the well-being attributes "calm", "relaxed", and "patient" are associated with a fifth well-being factor *F*₅ having a variance *v*₅;
- the variance *v*₁ is 48% of the sum of the variances of all well-being factors;
- the variance *v*₂ is 24% of the sum of the variances of all well-being factors;
- the variance *v*₃ is 12% of the sum of the variances of all well-being factors;
- the variance *v*₄ is 9% of the sum of the variances of all well-being factors; and
- the variance *v*₅ is 7% of the sum of the variances of all well-being factors.

It was further found that each attribute had a loading within each factor (*F₁, F₂, F₃, F₄, F₅*) as listed in Table 1

**Table 1**

| **well-being attribute** | **weight** | **well-being attribute** | **weight** |
|---|---|---|---|
| "not anxious" | 10.65% | "motivated" | 3.34% |
| "not sad" | 10.32% | "invigorated" | 3.31% |
| "not restless" | 9.84% | "interested" | 6.12% |
| "not frustrated" | 9.65% | "not bored" | 5.88% |
| "not stressed" | 7.54% | "not fatigued" | 4.59% |
| "happy" | 4.70% | "mentally alert" | 4.41% |
| "optimistic" | 4.61% | "calm" | 2.66% |
| "excited" | 4.13% | "relaxed" | 2.24% |
| "satisfied" | 3.91% | "patient" | 2.10% |

Using this methodology, the numerical results from the tests were summed to provide a mean for each attribute for each cream, and then these individual attribute scores were summed to provide an overall well-being score for each cream.

The overall well-being results are shown in Table 2:

**Table 2**

| **Overall Well-being** | | | | | |
|---|---|---|---|---|---|
| **Cream** | **time** | **#people** | **Well_Being** | **DeltaWell_Being** | **p-value*** |
| 4 | Before | 59 | 6.62767 | | |
| 4 | After | 59 | 7.03020 | .40253 | 0.0002 |
| 3 | Before | 59 | 6.78831 | | |
| 3 | After | 59 | 7.36640 | .57809 | <.0001 |

| | | | | | |
|---|---|---|---|---|---|
| *significant difference in average well-being between time points for this sample at 5% level (using per product ANOVA model). | | | | | |

The results for the positive well-being attributes (excited, happy, invigorated, motivated, optimistic, satisfied) before and after application are shown in Table 3

**Table 3**

| | | | **Positive Well-being Attribute Mean Scores - Factor 2** | | | | | |
|---|---|---|---|---|---|---|---|---|
| **Cream** | **time** | **#people** | **Excited** | **Happy** | **Invigorated** | **Motivated** | **Optimistic** | **Satisfied** |
| 4 | Before | 59 | 3.01695 | 6.87797 | 4.68644 | 6.52373 | 6.75763 | 6.57627 |
| 4 | After | 59 | 2.96610 | 7.01695 | 5.52542 | 6.66949 | 6.76271 | 6.58305 |
| 3 | Before | 59 | 3.71695 | 7.08475 | 5.15763 | 7.18814 | 6.99492 | 6.74068 |
| 3 | After | 59 | 3.28136 | 7.29153 | 6.42034 | 764407 | 7.52373 | 7.72712 |

| | | | | | | | | |
|---|---|---|---|---|---|---|---|---|
| Cream 3 has higher mean scores for all of the individual positive attributes, both before and after application | | | | | | | | |

The overall scores from Table 3 are summed in Table 4:

**Table 4**

| **Cream** | **time** | **#People** | **Factor 2** | **Delta Factor 2** | **Significance Tukey** |
|---|---|---|---|---|---|
| 4 | Before | 59 | 5.78873 | | |
| 4 | After | 59 | 5.94533 | 0.15660 | b |
| 3 | Before | 59 | 6.17936 | | |
| 3 | After | 59 | 6.64491 | 0.46555 | a |

| | | | | | |
|---|---|---|---|---|---|
| This shows that Cream 3 provides a significantly higher change in perception of well-being than does Cream 4 | | | | | |

The overall result was that, of the 59 test subjects, 46 experienced an enhanced feeling of well-being when tested with Cream 3, that is, 78% of the subjects experienced a feeling of enhanced well-being as a result of exposure to the cream containing the hyaluronic acid, as hereinabove described. The overall before and after scores for the test subjects with respect to cream 3 are in Table 5.

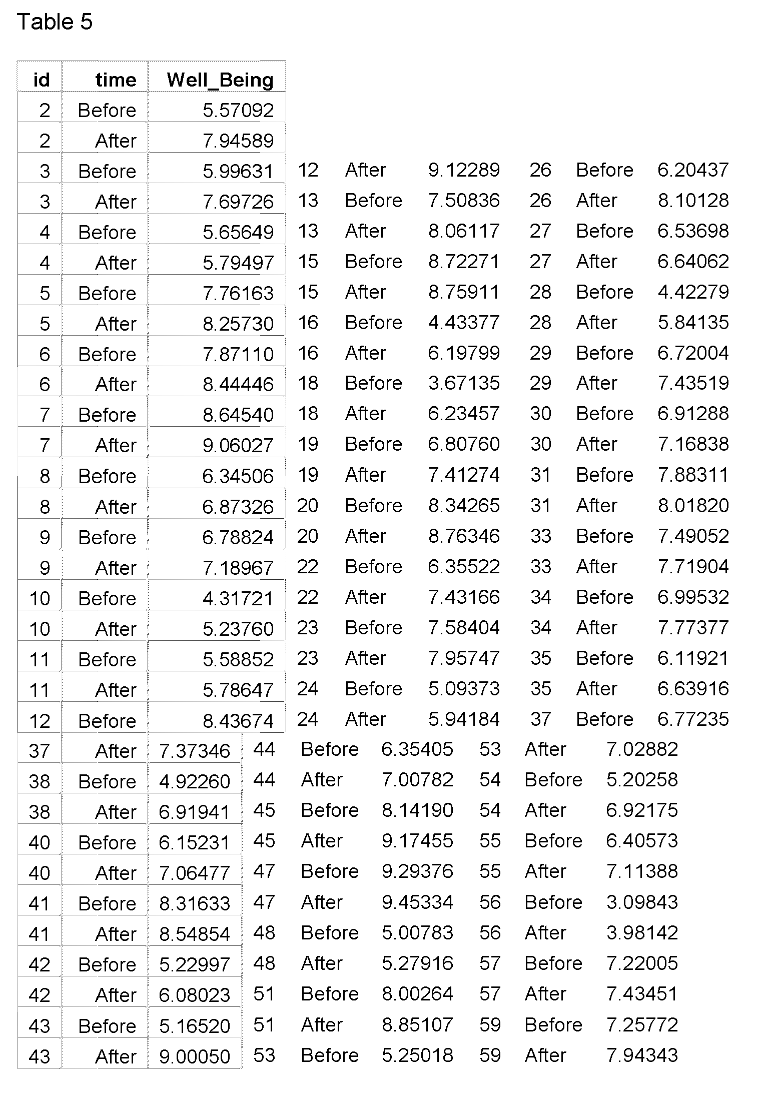

## Claims

1. A skin treatment preparation, comprising hyaluronic acid having a molecular weight of from 10-1000KDa, absorbed into an activated smectite clay by blending under conditions of high shear, the hyaluronic acid - clay blend having a non-pore specific surface of from 3-10 M²/g and a particle size (VSSA) of from 250-500nm.

2. A skin treatment preparation according to claim 1, in which the specific surface is from 4-9 M²/g and the particle size (VSSA) is from 280-45nm, particularly from 300-430nm

3. A skin treatment preparation according to claim 1, in which the specific surface is from 4-9 M²/g and the particle size (VSSA) is from 280-45nm, particularly from 300-430nm

4. A skin treatment preparation according to any one of claims 1-3, in which the molecular weight of the hyaluronic acid is from 20-1500KDa, particularly from 50-1400KDa, more particularly from 100-1100KDa, and even more particularly from 300-1000KDa.

5. A skin treatment preparation according to any one of claims 1-4, in which the activated smectite clay is montmorillonite, more particularly bentonite.

6. A skin treatment preparation according to any one of claims 1-5, in which the activated smectite clay is used in an unmodified form.

7. A skin treatment preparation according to any one of claims 1-5, in which the activated smectite clay is modified with a fatty substance.

8. A skin treatment preparation according to claim 7, in which the fatty substance is selected from the group consisting of oils and waxes of animal, vegetable or mineral origin, silicone oils, and mixtures thereof.

9. A method of providing hyaluronic acid to the skin, the hyaluronic acid comprising part of a hyaluronic acid-clay combination according to claim 1.

10. An activated smectite clay into which is blended hyaluronic acid under conditions of high shear, the hyaluronic acid - clay blend having a non-pore specific surface of from 3-10 M²/g and a particle size (VSSA) of from 250-500nm.

11. A method of preparing a smectite clay - hyaluronic acid blend according to claim 10, comprising the blending of the hyaluronic acid into the clay under conditions of high shear.

## Patentansprüche

1. Hautbehandlungspräparat umfassend Hyaluronsäure mit einem Molekulargewicht von 10-1000 kDa, absorbiert in einen aktivierten Smektit-Ton durch Mischen unter Bedingungen mit hoher Scherung, wobei das Hyaluronsäure-Ton-Gemisch eine spezifische Nichtporen-Oberfläche von 3-10 m²/g und eine Partikelgröße (VSSA) von 250-500 nm aufweist.

2. Hautbehandlungspräparat nach Anspruch 1, wobei die spezifische Oberfläche 4-9 m²/g und die Partikelgröße (VSSA) 280-45 nm, insbesondere 300-430 nm, beträgt.

3. Hautbehandlungspräparat nach Anspruch 1, wobei die spezifische Oberfläche 4-9 m²/g und die Partikelgröße (VSSA) 280-45 nm, insbesondere 300-430 nm, beträgt.

4. Hautbehandlungspräparat nach einem der Ansprüche 1-3, wobei das Molekulargewicht der Hyaluronsäure 20-1500 kDa, insbesondere 50-1400 kDa, weiter insbesondere 100-1100 kDa und speziell 300-1000 kDa beträgt.

5. Hautbehandlungspräparat nach einem der Ansprüche 1-4, wobei der aktivierte Smektit-Ton Montmorillonit, insbesondere Bentonit, ist.

6. Hautbehandlungspräparat nach einem der Ansprüche 1-5, wobei der aktivierte Smektit-Ton in einer unmodifizierten Form verwendet wird.

7. Hautbehandlungspräparat nach einem der Ansprüche 1-5, wobei der aktivierte Smektit-Ton mit einem Fettstoff modifiziert ist.

8. Hautbehandlungspräparat nach Anspruch 7, wobei der Fettstoff ausgewählt ist aus der Gruppe bestehend aus Ölen und Wachsen tierischen, pflanzlichen oder mineralischen Ursprungs, Siliconölen und Gemischen davon.

9. Verfahren zur Bereitstellung von Hyaluronsäure an die Haut, wobei die Hyaluronsäure einen Teil einer Hyaluronsäure-Ton-Kombination nach Anspruch 1 umfasst.

10. Aktivierter Smektit-Ton, dem Hyaluronsäure unter Bedingungen mit hoher Scherung beigemischt ist, wobei das Hyaluronsäure-Ton-Gemisch eine spezifische Nichtporen-Oberfläche von 3-10 m²/g und eine Partikelgröße (VSSA) von 250-500 nm aufweist.

11. Verfahren zur Herstellung eines Smektit-Ton-Hyaluronsäure-Gemischs nach Anspruch 10, umfassend Mischen der Hyaluronsäure in den Ton unter Bedingungen mit hoher Scherung.

## Revendications

1. Préparation pour le traitement de la peau, comprenant de l'acide hyaluronique ayant un poids moléculaire de 10 à 1 000 KDa, absorbé dans une argile de smectite activée par mélange dans des conditions de cisaillement élevé, le mélange acide hyaluronique - argile ayant une surface spécifique non poreuse de 3-10 M²/g et une taille de particule (VSSA) de 250 à 500 nm.

2. Préparation pour le traitement de la peau selon la revendication 1, dans laquelle la surface spécifique est de 4-9 M²/g et la taille de particule (VSSA) est de 280 à 45 nm, en particulier de 300 à 430 nm.

3. Préparation pour le traitement de la peau selon la revendication 1, dans laquelle la surface spécifique est de 4-9 M²/g et la taille de particule (VSSA) est de 280 à 45 nm, en particulier de 300 à 430 nm.

4. Préparation pour le traitement de la peau selon l'une quelconque des revendications 1 à 3, dans laquelle le poids moléculaire de l'acide hyaluronique est de 20 à 1 500 KDa, en particulier de 50 à 1 400 KDa, plus particulièrement de 100 à 1 100 KDa, et encore plus particulièrement de 300 à 1 000 KDa.

5. Préparation pour le traitement de la peau selon l'une quelconque des revendications 1 à 4, dans laquelle l'argile de smectite activée est la montmorillonite, plus particulièrement la bentonite.

6. Préparation pour le traitement de la peau selon l'une quelconque des revendications 1 à 5, dans laquelle l'argile de smectite activée est utilisée sous une forme non modifiée.

7. Préparation pour le traitement de la peau selon l'une quelconque des revendications 1 à 5, dans laquelle l'argile de smectite activée est modifiée avec une substance grasse.

8. Préparation pour le traitement de la peau selon la revendication 7, dans laquelle la substance grasse est choisie dans le groupe constitué par les huiles et cires d'origine animale, végétale ou minérale, les huiles de silicone et leurs mélanges.

9. Procédé d'administration d'acide hyaluronique à la peau, l'acide hyaluronique comprenant une partie d'une combinaison acide hyaluronique-argile selon la revendication 1.

10. Argile de smectite activée dans laquelle est mélangé de l'acide hyaluronique dans des conditions de cisaillement élevé, le mélange acide hyaluronique-argile ayant une surface spécifique non poreuse de 3-10 M²/g et une taille de particule (VSSA) de 250 à 500 nm.

11. Procédé de préparation d'un mélange argile de smectite-acide hyaluronique selon la revendication 10, comprenant le mélange de l'acide hyaluronique dans l'argile dans des conditions de cisaillement élevé.
